(19) Europäisches Patentamt
European Patent Office
Office européen des brevets

(11) **EP 4 546 349 A1**

(12) **EUROPEAN PATENT APPLICATION**

(43) Date of publication:
**30.04.2025 Bulletin 2025/18**

(21) Application number: **24208105.7**

(22) Date of filing: **22.10.2024**

(51) International Patent Classification (IPC):
**G16B 15/30** $^{(2019.01)}$  **G06N 3/045** $^{(2023.01)}$
**G16B 40/20** $^{(2019.01)}$  **G16B 40/30** $^{(2019.01)}$

(52) Cooperative Patent Classification (CPC):
**G16B 40/20; G06N 3/045; G16B 15/30; G16B 40/30**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **23.10.2023 KR 20230142142**

(71) Applicant: **LG Management Development Institute Seoul 07336 (KR)**

(72) Inventors:
• **Kim, Kiyoung**
  **07811 Seoul (KR)**
• **Kim, Youjin**
  **07796 Seoul (KR)**
• **Yim, Soorin**
  **07801 Seoul (KR)**
• **Hwang, Doyeong**
  **07524 Seoul (KR)**

(74) Representative: **BCKIP Part mbB MK1 Landsbergerstraße 98, 3.Stock 80339 München (DE)**

(54) **APPARATUS FOR A SEQUENCE TRANSFORM NEURAL NETWORK FOR TRANSFORMING AN INPUT SEQUENCE AND LEARNING METHOD USING THE SAME**

(57)    An apparatus for implementing a sequence transform neural network for transforming an input sequence having network inputs comprises a memory; a communicator; and a processor operably connected to the memory and the communicator The processor is configured to: receive first input data, receive second input data corresponding to the first input data, and generate output data predicting whether the first input data and the second input data are combined by further incorporating information corresponding to an experimental method into an embedding vector, determined by performing predetermined attention computation based the first input data and the second input data labeled on predetermined label information. The experimental method is comprised in one of a plurality of predetermined categories, and the experimental method and the experimental result data of the experimental method are grouped according to the predetermined categories and combined with the embedding vector to be used as an input feature.

**FIG. 2**

**Description**

**BACKGROUND**

The present disclosure generally relates to an apparatus and method for predicting a neoantigen. More specifically, some exemplary embodiments of the present disclosure relate to an apparatus for implementing a sequence transform neural network for transforming an input sequence and a learning method using the same, using as input features, by additionally combining an experimental method and experimental result data of the experimental method, each of which is classified into a plurality of categories for an embedded vector to be input to an artificial intelligence model.

A new kind of antigen composed of a protein complex is derived from somatic mutations, or like them, and may be formed by cancer cells and antigen-presenting cells of a subject. Immunogenicity means that T cells are activated by recognizing a protein complex to which major histocompatibility complex (MHC) proteins and peptide antigens are bound. In order for T cells to develop immunogenicity, the T cell receptor may be able to physically bind to the protein complex to which the MHC protein and the peptide antigen are bound.

Recently, the performance of various learning models for predicting an unknown structure of an MHC-peptide complex has been developed. However, due to noise, high cost, or the like which may occur during the learning process, it may be difficult to directly utilize the MHC-peptide complex structure in predicting binding with MHC peptide complex.

Therefore, there is an increasing need for a method that may improve the performance of a binding prediction model that predicts whether T cells bind to MHC-peptide complex using an artificial intelligence neural network. In addition, a learning approach that may more accurately predict the activity of a complex through a given MHC-peptide complex input may be needed.

In particular, when attempting to utilize the results of various experimental methods as input features in order to improve the efficiency of binding prediction for immunogenicity determination, there are several experimental methods, making the range very large, and for certain experimental methods, the amount of data is either too little or too much, thereby causing data imbalance depending on the experimental method and therefore it may be difficult to utilize all experimental data.

**[Prior Art Document]**

**[Patent Document]**

(Patent Document **0001**) Korean Patent Application Publication No. 10-2019-0177764 (published on December 30, 2019)

**SUMMARY**

Some embodiments of the present disclosure have been proposed to solve the above-described problems, and according to certain embodiments of the present disclosure, binding of the MHC-peptide complex may be predicted by dividing a plurality of experimental methods into a plurality of categories and utilizing them as input features in an embedded vector.

In addition, according to an embodiment of the present disclosure, a prediction device may perform data augmentation by using a causal relationship of each experimental result according to an experimental method to improve data imbalance and predict combination.

The problems to be solved by the present disclosure are not limited to those mentioned above, and other problems not mentioned may be clearly understood by those skilled in the art from the following description.

According to an embodiment of the present disclosure, an neural network implementation apparatus for implementing a sequence transform neural network for transforming an input sequence having respective network inputs by at least one computer according to the embodiment of the present disclosure, may include; a memory; a communication unit; and at least one processor electrically connected to the memory and the communication unit, wherein the at least one processor is configured to: receive first input data, receive second input data corresponding to the first input data, and form output data predicting whether the first input data and the second input data are combined by further incorporating information corresponding to the experimental method into an embedding vector, which is determined through performing a predetermined attention computation based the first input data and the second input data labeled on predetermined label information, the experimental method being comprised in one of a plurality of predetermined categories, and the experimental method and the experimental result data of the experimental method being grouped according to the plurality of predetermined categories and combined with the embedding vector to be used as an input feature.

A learning method for transforming an input sequence, performed by at least one processor of a computer device, according to an embodiment of the present disclosure, may include: receiving first input data; receiving second input data corresponding to the first input data; determining an embedding vector by performing a predetermined attention computation based the first input data and the second input data labeled on predetermined label information; further combining, the experimental method and the experimental result data regarding whether the first input data and the second input data are combined, into determined embedding vector, by grouping according to a plurality of the predetermined categories; forming output data predicting whether the first input data and the second input data are combined, by using an embedding vector further combined with information corresponding to the experimental method as an input feature.

In addition, other method and system for implementing the present disclosure and a computer-readable recording medium storing a computer program for executing the method, may further be provided.

Additionally, a computer program stored on a medium, allowing the method of implementing the present disclosure to be performed on a computer, may further be provided.

According to certain embodiments of the present disclosure, it is possible to improve prediction accuracy by classifying a large number of experimental methods into a more limited number of categories, and performing binding prediction learning of the MHC-peptide complex while maintaining data balance.

In addition, according to some embodiments of the present disclosure, it is possible to improve prediction accuracy by estimating experimental result data from another experimental stage based on experimental result data from one experimental stage and performing binding prediction learning of the MHC-peptide complex through data enhancement.

The effects of the present disclosure are not limited to the effects mentioned above, and other effects not mentioned may be clearly understood by those skilled in the art from the following description.

## BRIEF DESCRIPTION OF DRAWINGS

FIG. 1 is a schematic block diagram of a neural network implementation apparatus for implementing a sequence transform neural network according to various embodiments of the present disclosure.

FIG. 2 is a diagram schematically illustrating a learning operation for implementing a sequence transform neural network according to various embodiments of the present disclosure.

FIG. 3 is a diagram schematically illustrating an attention algorithm of a sequence transform neural network according to various embodiments of the present disclosure.

FIG. 4 is a diagram illustrating an example of generating output data by using attention score values calculated from attention algorithms of sequence transform neural networks according to various embodiments of the present disclosure.

FIG. 5 is a diagram illustrating a plurality of categories of experimental methods and a plurality of types of experimental methods belonging to the plurality of categories to be additionally combined with an embedded vector of a sequence transform neural network according to various embodiments of the present disclosure.

FIG. 6 is a diagram illustrating an input feature in which information corresponding to an experimental method is additionally combined with an embedded vector of a sequence transform neural network according to various embodiments of the present disclosure.

FIG. 7 is a diagram illustrating an example of performing a scaled dot attention operation in an attention algorithm of a sequence transform neural network according to various embodiments of the present disclosure.

FIG. 8 is a conceptual diagram illustrating an example of processing second input data in an attention algorithm of a sequence transform neural network according to various embodiments of the present disclosure.

FIG. 9 is a conceptual diagram illustrating an example of processing first input data in an attention algorithm of a sequence transform neural network according to various embodiments of the present disclosure.

FIG. 10 is a diagram illustrating a process in which a sequence transform neural network performs data enhancement by using an association relationship between each step of an experimental method according to various embodiments of the present disclosure.

FIG. 11 is a flow-chart of a learning method for transforming an input sequence according to various embodiments of the present disclosure.

**DETAILED DESCIPTION**

Throughout the present disclosure, the same reference numerals designate the same components. The present disclosure does not describe all elements of the embodiments, and general contents in the technical field of the present disclosure or duplicated content among the embodiments are omitted. The terms "unit, module, member, block" used in the specification may be implemented in software or hardware, and depending on the embodiments, multiple "units, modules, members, blocks" may be implemented as a single component, or a single "unit, module, member, block" may also include multiple components.

Throughout the specification, when a part is described as being "connected" to another part, it includes not only cases where they are directly connected but also cases where they are indirectly connected, wherein indirectly connect may include cases where they are connected by a wireless communication network.

In addition, when a part is described as "comprising" a certain component, it means that it may further include other components, not excluding other components unless explicitly stated otherwise.

Throughout the specification, when one member is described as being "on" other member, it includes not only cases where the members are in contact but also cases where another member exists between them.

Terms such as "first" and "second" are used to distinguish one component from another, and are not intended to limit the components to those aforementioned by the terms.

Singular expressions include plural expressions unless the context clearly indicates otherwise.

Identification codes used for each step are provided for convenience in description and do not specify the order of the steps, and the each step may be carried out in a different order unless a specific order is explicitly described.

The operating principles and embodiments of the present disclosure will be described below with reference to the accompanying drawings.

The term "device according to the present disclosure" in this specification encompasses various devices capable of providing results to users by performing operations. For example, the device according to the present disclosure may include a computer, server device, and portable terminal, or it may take any one of these forms.

Here, the computer may include, for example, a notebook, desktop, laptop, tablet personal computer (PC), slate PC, or like them equipped with a web browser.

The server device is a server that processes information by communicating with an external device, and may include an application server, a computing server, a database server, a file server, a game server, a mail server, a proxy server, a web server, and like them.

The portable terminal is for example, a wireless communication device ensuring portability and mobility, and may include all kinds of handheld-based wireless communication devices such as Personal Communication System (PCS), Global System for Mobile communications (GSM), Personal Digital Cellular (PDC), Personal Handyphone System (PHS), Personal Digital Assistant (PDA), International Mobile Telecommunication (IMT)-2000, Code Division Multiple Access (CDMA)-2000, W-Code Division Multiple Access (W-CDMA), Wireless Broadband Internet (WiBro) terminal, smart phone, or like them, and wearable devices such as watches, rings, bracelets, anklets, necklaces, glasses, contact lenses, head-mounted devices (HMD), or like them.

The term "antigen" as used herein may refer to a substance that induces an immune response.

A neoantigen may refer to a new protein formed in cancer cells when a specific mutation occurs in tumor deoxyribonucleic acid (DNA).

The neoantigen are generated by mutations, and are expressed only in cancer cells.

The neoantigen may comprise a polypeptide sequence or a nucleotide sequence. Mutations may include frameshift or non-lattice shift indels, missense or nonsense substitutions, splice site alterations, genomic rearrangements or gene fusions, or any genomic or expression alteration that leads to the creation of a novel open reading frame (ORF). The mutation may also include a splice variant. Post-translational modifications specific to tumor cells may include abnormal phosphorylation. Post-translational modifications specific to tumor cells may also include proteasome-generated spliced antigens.

The term "Epitope" may refer to the specific part of an antigen to which an antibody or T-cell receptor typically binds.

The term "MHC" may refer to a protein that allows T cells to recognize the cell, by presenting a 'peptide' synthesized in a specific cell on the cell surface.

The term "peptide" may refer to a polymer of amino acids. For convenience of explanation, hereinafter, "peptide" refers to an amino acid polymer to an amino acid sequence represented by a cancer cell on a surface.

The term "MHC-peptide complex" is expressed on the surface of cancer cells, and is a complex structure of MHC and a peptide. T-cells recognize MHC-peptide complexes to perform immune responses.

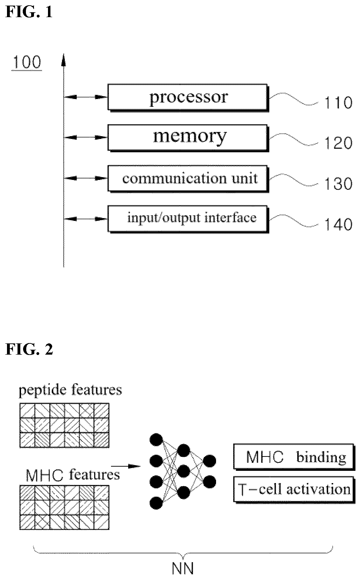

FIG. 1 is a schematic block diagram of a neural network implementation apparatus for implementing a sequence transform neural network according to various embodiments of the present disclosure. Hereinafter, an apparatus for implementing a sequence transform neural network for transforming an input sequence and a learning method using the same will be described with reference to FIGS. 1 to 11.

A sequence transform neural network 100 may include one or more processors 110, memory 120, a communicator or communication unit 130, an input and output interface 140, and the like. However, these internal components included in the sequence transform neural network 100 are provided for illustration purposes only, although not limited thereto. Alternatively or additionally, the sequence transform neural network 100 according to an embodiment of the present disclosure may perform the functions of the processor 110 through a separate processing server or cloud server instead of a processor.

Referring to FIG. 1, the processor 110 may be configured to perform one or more operations of the sequence transform neural network 100 in association with the memory 120 that stores data on an algorithm for controlling the operations of components included in or associated with the sequence transform neural network 100 or a program that reproduces the algorithm, and the data stored in the memory 120. For example, the processor 110 and memory 120 may be implemented as separate chips. Alternatively, the processor 110 and the memory 120 may be implemented as a integrated single chip.

The memory 120 according to an embodiment may store data performing or supporting various functions of the sequence transform neural network 100, programs for the operations of the processor 110, input and/or output data (e.g., images, videos, etc.), multiple application programs or applications running on the sequence transform neural network 100, and data or instructions for operating the sequence transform neural network 100. At least some part of these application programs may be downloaded from an external server via wired or wireless communication.

The memory 120 may include at least one type of storage medium, such as flash memory type, hard disk type, Solid State Disk (SSD) type, Silicon Disk Drive (SDD) type, multimedia card micro type, card type memory (e.g., SD or XD memory), random access memory (RAM), static random access memory (SRAM), read-only memory (ROM), electrically erasable programmable read-only memory (EEPROM), programmable read-only memory (PROM), magnetic memory, magnetic disk, and optical disk. Additionally, the memory may be a database that is separate from the sequence transform neural network 100, but may be connected wired or wirelessly.

The communicator or communication unit 130 according to an embodiment may include one or more components configured to communicate with external devices, for example, at least one of a broadcast receiving module or broadcast receiver, wired communication module or wired communicator, wireless communication module or wireless communicator, short-range communication module or short-range communicator, or location information module.

The wired communication module may include various wired communication modules such as a Local Area Network (LAN) module, a Wide Area Network (WAN) module, or a Value Added Network (VAN) module, as well as various cable communication modules such as Universal Serial Bus (USB), High Definition Multimedia Interface (HDMI), Digital Visual Interface (DVI), recommended standard 232 (RS-232), power line communication, or plain old telephone service (POTS).

The wireless communication module may include not only a WiFi module, a Wireless broadband (WiBro) module, but also a wireless communication module supporting various wireless communication methods such as Global System for Mobile Communication (GSM), Code Division Multiple Access (CDMA), Wideband Code Division Multiple Access (WCDMA), Universal Mobile Telecommunications System (UMTS), Time Division Multiple Access (TDMA), Long Term Evolution (LTE), 4G (Generation), 5G, and 6G.

The short-range communication module is for short-range communication, and may support short-range communication by using at least one of following technologies: Bluetooth™, Radio Frequency Identification (RFID), Infrared Data Association (IrDA), Ultra Wideband (UWB), ZigBee, Near Field Communication (NFC), Wireless-Fidelity (Wi-Fi), Wi-Fi Direct, or Wireless Universal Serial Bus (Wireless USB).

The input and/or output interface 140 according to an embodiment serves as a channel for various types of external devices connecting to the sequence transform neural network 100. The input and/or output interface 140 may include, for example, but not limited to, at least one of the following: a wired and/or wireless headset port, an external charger port, a wired and/or wireless data port, a memory card port, a port for connecting a device equipped with a Subscriber Identification Module (SIM), an audio Input/Output (I/O) port, a video Input/Output (I/O) port, or an earphone port. The sequence transform neural network 100 may perform control related to the external device connected to the input and/or output interface 140.

Each module or component shown in FIG. 1 represents software and/or hardware components such as a Field Programmable Gate Array (FPGA) and an Application Specific Integrated Circuit (ASIC).

Referring to FIG. 2, pre-stored open data may be used when learning a sequence transform neural network (NN) according to an embodiment of the present disclosure.

For example, the MHC feature 110 is input as first input data, and the peptide feature 120 is input as second input data, each being input as respective input data so that whether MHC binding is possible and whether T-cell activation is possible can be learned.

As a result, according to some exemplary embodiments of the present disclosure, a sequence transform neural network NN of FIG. 2 for transforming an input sequence may be implemented using the sequence transform neural network 100 illustrated in FIG. 1.

Here, the pre-stored open data used for the learning may include data regarding MHC binding and T-cell activation according to the MHC type of each peptide. Detailed descriptions related thereto will be described later.

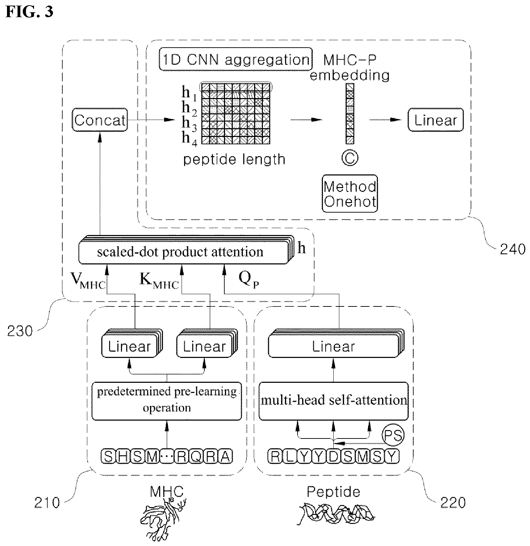

Referring to FIG. 3, when first input data is input, a computer device, computer or processor performs one or more operations of predetermined pre-learning to determine a first key and a first bell value for the first input data 210, and when second input data corresponding to the first input data are input, performs one or more operations of multi-head self-attention to generate a first query for the first input data 220.

Next, a scaled dot product attention operation is performed based on a first key, a first value, and a first query, and a matrix in which each sequence is transformed into a vector is output by concatenating each attention head 230. Next, a convolutional neural network (CNN) merging operation according to the peptide length is performed, an embedding operation is performed to transform into vectors, and output data is generated and output through a linear layer in 240.

In this exemplary embodiment, the output data may be obtained by extracting (e.g. filtering) only at least one MHC candidate or MHC-peptide complex candidate capable of binding and activating T-cells.

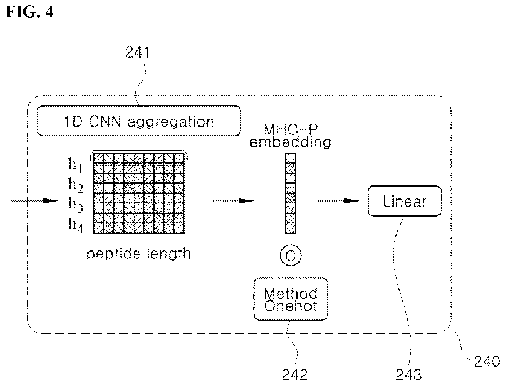

FIG. 4 is a diagram illustrating an example of generating output data by using an attention score value calculated by an attention algorithm of a sequence transform neural network implemented according to an embodiment of the present disclosure. A processing operation in 240 of FIG. 3 will be specifically described below.

Referring to FIG. 4, when a matrix in which each sequence is transformed into a vector is output by 230 in FIG. 3, the computer device performs a CNN merging operation according to the peptide length (241), and performs embedding to transform into a vector (242). For instance, the computer device generates an embedding vector for the first input data and the second input data. Next, output data is generated and output through a linear layer (243). At this time, information corresponding to the experimental method may be further combined with the embedding vector using one-hot encoding to generate output data.

According to one embodiment of the present disclosure, first input data is received, second input data corresponding to the first input data is received, and information corresponding to the experimental method is further combined into a determined embedding vector by performing a predetermined attention computation based on the predetermined label information and the labeled first and second input data, thereby forming output data that predicts whether the first input data and the second input data are bound.

In this embodiment, the experimental method is included in one of a plurality of predetermined categories, and the experimental method and experimental result data of the experimental method may be grouped according to a plurality of the predetermined categories and combined with the embedding vector, thereby being used as an input feature.

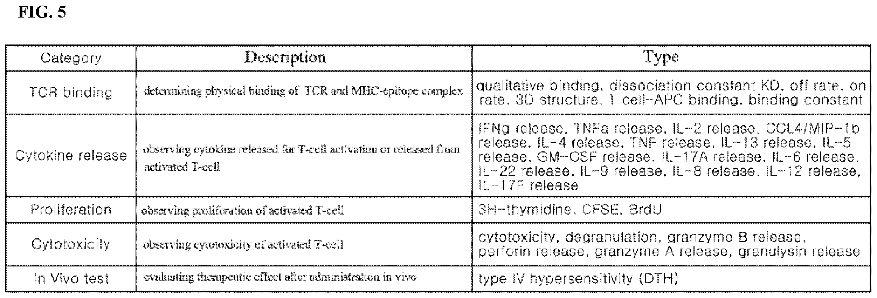

Referring to FIG. 5, the experimental method additionally combined with the one-hot encoding is classified into a corresponding category.

As an example, the category may be T-cell receptor (TCR) binding, Cytokine release, Proliferation, Cytotoxicity, In Vivo test.

The TCR binding is a category of experimental methods for determining whether a T cell receptor binds to an antigen-specific antigen. The TCR binding indicates immunogenicity if the experimental results of the experimental methods belonging to the TCR binding are binding (positive), and the TCR binding indicates no immunogenicity if there is no binding (negative). Only positive experimental results among the experimental result data of the TCR binding may be used as learning data.

As an example, the experimental method of the TCR binding may measure the physical binding of the TCR and the MHC-epitope complex. For example, the TCR binding may include experimental methods such as qualitative binding, dissociation constant (Kd), off rate, on rate, 3D structure, T cell-antigen presenting cell (APC) binding, or binding constant.

Cytokine release is a category of experimental methods for measuring whether T cells release cytokines in response to antigen-specific antigens. The Cytokine release indicates immunogenicity if the experimental results of the experimental methods belonging to cytokine release are cytokine releasing (positive), and the Cytokine release indicates no immunogenicity if there is no cytokine releasing (negative). Only positive experimental results among the experimental result data of the Cytokine release may be used as the learning data.

As an example, an experimental method of the cytokine release may observe cytokines released for T cell activation or released from activated T cells. For example, the Cytokine release may include experimental methods such as IFNg release, TNFa release, IL-2 release, CCL4/MIP-1b release, IL-4 release, TNF release, IL-13 release, IL-5 release, GM-CSF release, IL-17A release, IL-6 release, IL-22 release, IL-9 release, IL-8 release, IL-12 release, and IL-17F release.

Proliferation is a category of experimental methods for determining whether T cells proliferate in response to antigen-specific antigens. The proliferation indicates immunogenicity if the experimental result of an experimental method belonging to proliferation shows proliferation above a predetermined threshold (positive), and the proliferation indicates no immunogenicity if there is no proliferation above the predetermined threshold (negative). Both positive and negative experimental results among the experimental result data of proliferation may be used as the learning data.

As an example, the experimental method of the proliferation may observe the proliferation of activated T cells. For example, the proliferation may include experimental methods such as 3H-thymidine, carboxyfluorescein succinimidyl ester (CFSE), and Bromodeoxyuridine (BrdU).

Cytotoxicity is a category of experimental methods for determining whether T cells have the ability to kill antigen-specific antigens. The cytotoxicity indicates immunogenicity if the experimental result of an experimental method belonging to the cytotoxicity shows toxicity (positive), and the cytotoxicity indicates no immunogenicity if no toxicity (negative). Both positive and negative experimental results among the experimental result data of cytotoxicity may be used as the learning data.

As an example, the experimental method of the cytotoxicity may observe cytotoxicity of activated T cells. For example, the cytotoxicity may include experimental methods such as cytotoxicity, degranulation, granzyme B release, perforin release, granzyme A release, and granulysin release.

In Vivo test is a category of experimental methods for determining whether T cells have the ability to remove antigen-specific antigens in the body. The In Vivo test indicates immunogenicity if the experimental result of an experimental method belonging to the In Vivo test shows therapeutic efficacy (positive), and the In Vivo test indicates no immunogenicity if there is no therapeutic efficacy (negative). Both positive and negative experimental results among the experimental result data of the In Vivo test may be used as the learning data.

As an example, the experimental method of the In Vivo test may evaluate the therapeutic effect after administration. For example, the In Vivo test may include experimental methods such as type IV hypersensitivity (DTH).

Thus, the experimental method grouped into the above-described exemplary categories and the experimental result data of the experimental method may be combined with the MHC-P embedding vector by one-hot encoding, and input to the linear layer.

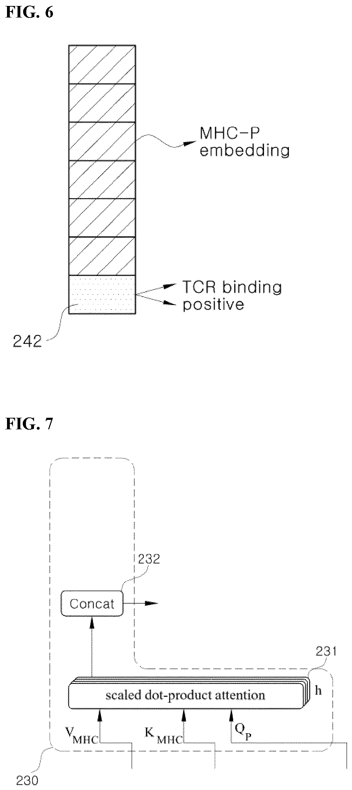

Referring to FIG. 6, it may be understood that the experimental method and the experimental result data of the experimental method that are categorized in the MHC-P embedding vector output according to the processing operations in 210, 220, and 230 of FIG. 3 to be described later are further combined into vectors 242.

For example, positive experimental result data of qualitative binding may be summarized and further combined into the categories TCR binding and positive. Through this, it is possible to efficiently generate learning data by solving the problem of imbalance of experimental result data between experimental methods and adding experimental methods in a category.

FIG. 7 is a diagram illustrating an example of performing scaled dot product attention in an attention algorithm of a sequence transform neural network implemented according to an embodiment of the present disclosure. A processing operation in 230 of FIG. 3 will be specifically described below.

Referring to FIG. 7, self-attention may be performed based on the first key, the first value, and the first query of the first input data. An attention score value is output as the output value at 231, and a plurality or all of attention heads thereof are concatenated at 232.

Here, the self-attention is for extracting relationship between the three elements which are keys, values and queries, and may refer to a scaled dot product attention operation.

In this case, the computer device calculates a first query for each sequence of the first input data as an attention score value for all first keys, and obtains a probability distribution in which all values are summed to be 1 by applying a softmax function.

This operation is referred to as attention distribution, and each value is referred to as an attention weight value.

The computer device may calculate an attention value by weighting the attention weight value and the hidden state for each sequence, and concatenate the attention value with the hidden state at time t to form a single vector.

That is, the computer device may perform an operation to sum a plurality of attention heads formed through a predetermined operation based on the first key, the first value, and the first query corresponding to each sequence of the first input data and the second input data(e.g., the softmax function).

At this time, the attention weight value corresponds to attention energy for the second input data corresponding to the first input data.

As a result, an attention value matrix having both attention values for each of the sequences of the first input data and the second input data is calculated. This may be represented by the following Equation 1:

[Equation 1]

$$\text{Attention}(Q,\ K,\ V)\ =\ \text{softmax}(\frac{QK^T}{\sqrt{d_k}})V$$

Equation 1 represents the attention operation, Q represents a query for performing an attention operation, K represents a key, and V represents a value corresponding to the query and the key.

The softmax function is an activation function used in multiple classes, and may mean a function that receives an N-dimensional vector when there are N classes and estimates the probability of belonging to each class (N is a natural number). The softmax function may be defined by Equation 2:

[Equation 2]

$$y_k = \frac{e^{a_k}}{\sum_{i=1}^{n} e^{a_i}}$$

Equation 2 may refer to a softmax function, n may refer to the number of neurons in the output layer, and k may refer to the order of classes.

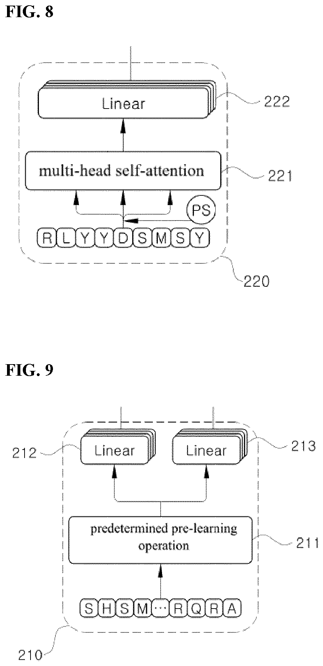

On the other hand, the first key and the first value for the first input data are determined from the first input data. However, the first query may be generated from the second input data, which will be specifically described below based on FIGS. 8 to 9.

On the other hand, the output data may be implemented as data indicating a degree of matching between sequences according to an attention score value calculated by performing self-attention in an attention algorithm of a sequence transform neural network.

Specifically, the output data may be formed by forming the output data in which T-cell immunogenicity is matched with each of sequence of the output data corresponding to the first input data, and performing a normalization operation on the T-cell immunogenicity corresponding to the sequence of the output data to form label information corresponding to the second input data.

FIG. 8 is a conceptual diagram illustrating an example of processing second input data in an attention algorithm of a sequence transform neural network implemented according to an embodiment of the present disclosure. The processing operation in 220 of FIG. 3 will be specifically described below.

Referring to FIG. 8, when the second input data corresponding to the first input data is input to the implemented sequence transform neural network, the computer device matches the positional information with each of sequence of the second input data. For instance, the second input data is a peptide feature (sequence), and both a physicochemical property (AAindex) and an amino acid substitution matrix (BLOSUM) are used as the peptide feature. As an example, the second input data may be composed of 9-10 sequences.

Subsequently, at 221, the computer device may generate a second key, a second value, and a second query corresponding to the second input data, that is, for each sequence of the second input data, by calculating with the attention weight through the input embedding, and perform the predetermined self-attention operation based on the second key, the second value and the second query. The attention weight may be calculated through pre-learning.

Here, self-attention is multi-head attention, and may be performed by performing num_heads parallel attentions on a second key, a second value and a second query having a dimension obtained by dividing a dimension of a $d_{model}$ by num_head, and concatenating all attention heads (each attention value matrix) thereof.

Different attention weights WQ, WK, and WV are given to each attention head. Then, a value obtained by multiplying a matrix to which all of the attention heads are concatenated by another weight matrix (WO) is output as a final result value for multi-head attention.

At 222, the output values are then passed through a linear layer to generate the first query for the first input data.

FIG. 9 is a conceptual diagram illustrating an example of processing first input data in an attention algorithm of a sequence transform neural network implemented according to an embodiment of the present disclosure. The processing operation in 210 of FIG. 3 will be specifically described below.

Referring to FIG. 9, when the first input data is input to the implemented sequence transform neural network, the computer device performs a predetermined pre-learning operation at 211, and determines a first key and a first value by passing respective output values thereof through a linear layer at 212 and 213.

For example, the first input data may include at least one of the MHC type and the MHC structure information.

Specifically, the first input data is an MHC feature (a three-dimensional structure), and 181 sequences close to the binding site may be used for the MHC feature, and as an example, the MHC type may be changed to 360 sequences. Meanwhile, the first input data may be provided as a sequence corresponding to a predetermined range with reference to a point at which the peptide sequence and the MHC sequence bind.

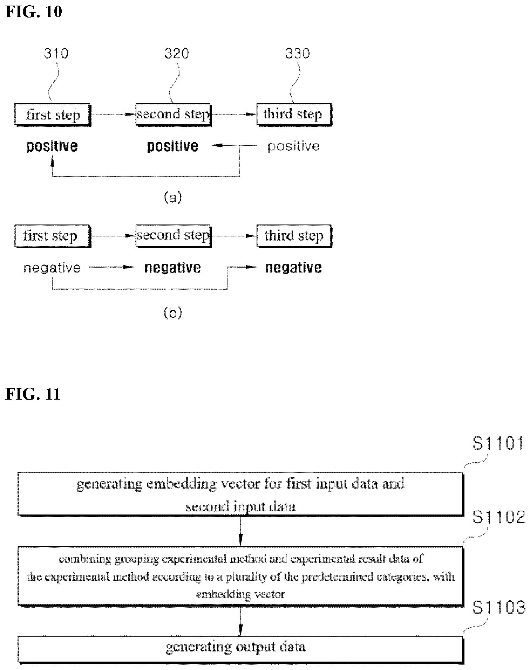

On the other hand, as shown in FIG. 10, data enhancement may be performed using experimental result data of each experimental method.

As an example, if the experimental method comprises a plurality of experimental steps, the experimental results for other experimental stages are estimated based on the experimental result data for each experimental stage, thereby performing the learning through data augmentation.

Referring to FIG. 10, in the case where the experimental method comprises a first step 310, a second step 320, and a third step 330, and the experimental result data in the third step 330 is positive, then the experimental result data of the first step 310 and the second step 320 may be estimated to be positive.

Alternatively, in the case where the experimental method includes a first step 310, a second step 320, and a third step 330, the experimental result data of the first step 310 is negative, then the experimental result data in the second step 320 and the third step 330 may be estimated to be negative.

For example, in the case of an experimental method that goes through a perforin, granzyme B release step, a degranulation step, and a cytotoxic effect step, in order for the cytotoxic effect step to be positive, both the perforin, the granzyme B released step and the degranulation step must be positive.

Accordingly, when the experimental result data of the cytotoxic effect step is positive, the experimental result data for the perforin, granzyme B release step, and degranulation step may be positive to further generate the experimental method and the experimental result data.

In addition, when the experimental result data of the perforin, granzyme B release step is negative, the experimental result data for the degranulation step and the cytotoxic effect step may only be negative.

Therefore, when the experimental result data of the perforin, granzyme B release step is negative, the experimental result data for the degranulation step and the cytotoxic effect step may be negative to further generate the experimental method and the experimental result data.

The amount of learning data may be adjusted by performing data enhancement using a positive set and a negative set of Cytokine.

As an example, for a category including an experimental method in which a data amount is insufficient among a plurality of categories, experimental results for other experimental steps may be estimated based on experimental result data for each experimental step, thereby performing data enhancement.

According to some embodiments of the present disclosure, it is possible to solve the problem of learning data imbalance and improve learning efficiency by determining whether to perform data enhancement based on the amount of experimental result data of the experimental method.

In addition, the pre-set specific category among the plurality of categories may perform learning only by an experimental method in which experimental result data is positive and experimental result data of the experimental method.

As an example, the experimental method of TCR binding and cytokine release may perform learning only with data that has experimental result data is positive. On the other hand, the experimental method of Proliferation, Cytotoxicity, and In Vivo test may perform learning using the experimental result data, whether positive or negative.

The above-described learning data may be used to perform a learning method for transforming an input sequence, which is performed by a sequence transform neural network on a computer device.

The first input data and the second input data corresponding to the first input data may be input to perform a learning method.

Referring to FIG. 11, an embedding vector for the first input data and the second input data may be generated at operation S1101. The embedding vector may be determined by performing a predetermined attention computation based on the first input data and the second input data labeled on predetermined label information.

At operation S1102, the experimental method and the experimental result data of the experimental method may be grouped into a plurality of predetermined categories and combined with the embedding vector. The experimental method of whether the first input data and the second input data are combined and the experimental result data of the experimental method may be further combined into an embedding vector determined by grouping according to the predetermined plurality of categories.

Then, at operation S1103, output data may be generated. The embedding vector to which information corresponding to the experimental method is additionally combined may be used as an input feature to form output data for predicting whether the first input data and the second input data are combined.

Meanwhile, some of the disclosed embodiments may be implemented in the form of a recording medium that stores instructions executable by a computer. The instructions may be stored in the form of program code and, when executed by a processor, may generate program modules to perform the operations of the disclosed embodiments. The recording medium may be implemented as a computer-readable recording medium.

The computer-readable recording medium includes any type of recording medium in which instructions that may be decrypted by a computer are stored. For example, Examples include a read only memory (ROM), a random access memory (RAM), a magnetic tape, a magnetic disk, a flash memory, an optical data storage device, and the like.

As described above, the disclosed embodiments are described with reference to the accompanying figures. A person of ordinary skill in the art may understand that the present disclosure may be implemented in a different form from the disclosed embodiments without changing the technical sprit or essential features of the present disclosure. The disclosed embodiments are illustrative and restrictive.

**[Explanation of Symbols]**

100: sequence transform neural network

110: processor

120: memory

130: communication unit

140: input/output interface

310, 320, 330: experimental method stages

**Claims**

1. An apparatus for a sequence transform neural network for transforming an input sequence having network inputs, the apparatus comprising:

   a memory;
   a communicator; and
   at least one processor operably connected to the memory and the communicator,
   wherein the at least one processor is configured to:

   receive first input data,
   receive second input data corresponding to the first input data, and
   generate output data predicting whether the first input data and the second input data are combined, by incorporating information corresponding to an experimental method into an embedding vector, which is determined by performing predetermined attention computation based on the first input data and the second input data labeled on predetermined label information,
   wherein:
   the experimental method is comprised in one of a plurality of predetermined categories, and
   the experimental method and experimental result data of the experimental method are grouped according to the plurality of predetermined categories and combined with the embedding vector to be an input feature.

2. The apparatus of claim 1, wherein: the at least one processor is configured to:

   perform predetermined pre-training computation based on the first input data to determine a first key and a first value,
   match positional information to each sequence of the second input data, and
   generate a first query by performing predetermined self-attention computation based on a second key, a second value, and a second query corresponding to the second input data, and
   combine the experimental method and the experimental result data included in the plurality of categories with the embedding vector determined by performing the predetermined attention computation based on the first key, the first value, and the first query to be the input feature.

3. The apparatus of claim 2, wherein:
   the plurality of categories comprise T-cell receptor (TCR) binding, Cytokine release, Proliferation, Cytotoxicity, and In Vivo test.

4. The apparatus of claim 3, wherein:

the first input data comprises at least one of major histocompatibility complex (MHC) type and MHC structural information, and

the second input data comprises a plurality of peptide sequences.

5. The apparatus of claim 1, wherein:
the at least one processor is configured to, when the experimental method comprises multiple experimental stages, based on experimental result data for each stage, perform learning through data augmentation by estimating experimental results of other experimental stages.

6. The apparatus of Claim 5, wherein:
the at least one processor is configured to perform the data augmentation by estimating the experimental results for the other experimental stages based on the experimental result data for each stage for a category that contains experimental methods with insufficient data among the plurality of categories.

7. The apparatus of claim 6, wherein:

the experimental method comprises a first stage, a second stage, and a third stage, and
the at least one processor is configured to, if experimental result data of the third stage is positive, estimate experimental result data of the first stage and the second stage as positive.

8. The apparatus of claim 7, wherein:
the at least one processor is configured to, if the experimental result data of the first stage is negative, estimate the experimental result data of the second stage and the third stage as negative.

9. The apparatus of claim 8, wherein:
the at least one processor is configured to perform an operation in which, when the experimental result data are positive, a pre-set specific category among the plurality of categories performs the learning by only the experimental method and the experimental result data of the experimental method.

10. A learning method for a sequence transform neural network, comprising:

receiving first input data;
receiving second input data corresponding to the first input data;
determining an embedding vector by performing predetermined attention computation based on the first input data and the second input data labeled on predetermined label information;
combining experimental method and experimental result data, regarding whether the first input data and the second input data are combined, with the determined embedding vector, by grouping according to a plurality of the predetermined categories;
generating output data predicting whether the first input data and the second input data are combined, by using the embedding vector combined with information corresponding to the experimental method as an input feature.

11. The learning method of claim 10, wherein:
the combining of the experimental method and the experimental result data comprises:

determining a first key and a first value by performing predetermined pre-training computation based on the first input data;
matching positional information to each sequence of the second input data;
generating a first query by performing predetermined self-attention computation based on a second key, a second value, and a second query corresponding to the second input data; and
combining the experimental method and the experimental result data included in the plurality of categories with the embedding vector determined by performing the predetermined attention computation based on the first key, the first value, and the first query to be the input feature.

12. The learning method of claim 11, wherein:

the plurality of categories comprise T-cell receptor (TCR) binding, Cytokine release, Proliferation, Cytotoxicity, and In Vivo test,
the first input data comprises at least one of major histocompatibility complex (MHC) type and MHC structural information, and
the second input data comprises a plurality of peptide sequences.

**13.** The learning method of claim 10, further comprising:

when the experimental method comprises multiple experimental stages, based on experimental result data for each stage, performing learning through data augmentation by estimating experimental results of other experimental stages; and
performing the data augmentation by estimating the experimental results of the other experimental stages based on the experimental result data for each stage for a category that contains experimental methods with insufficient data among the plurality of categories.

**14.** The learning method of claim 13, wherein:
the experimental method comprises a first stage, a second stage, and a third stage, further comprising:
the learning method further comprises:

if experimental result data of the third stage is positive, estimating experimental result data of the first stage and the second stage as positive; and
if the experimental result data of the first stage is negative, estimating the experimental result data of the second stage and the third stage as negative.

**15.** The learning method of claim 14, wherein:
when the experimental result data are positive, a pre-set specific category among the plurality of categories performs the learning by only the experimental method and the experimental result data of the experimental method.

**FIG. 1**

**FIG. 2**

**FIG. 3**

**FIG. 4**

**FIG. 5**

| Category | Description | Type |
|---|---|---|
| TCR binding | determining physical binding of TCR and MHC-epitope complex | qualitative binding, dissociation constant KD, off rate, on rate, 3D structure, T cell-APC binding, binding constant |
| Cytokine release | observing cytokine released for T-cell activation or released from activated T-cell | IFNg release, TNFa release, IL-2 release, CCL4/MIP-1b release, IL-4 release, TNF release, IL-13 release, IL-5 release, GM-CSF release, IL-17A release, IL-6 release, IL-22 release, IL-9 release, IL-8 release, IL-12 release, IL-17F release |
| Proliferation | observing proliferation of activated T-cell | 3H-thymidine, CFSE, BrdU |
| Cytotoxicity | observing cytotoxicity of activated T-cell | cytotoxicity, degranulation, granzyme B release, perforin release, granzyme A release, granulysin release |
| In Vivo test | evaluating therapeutic effect after administration in vivo | type IV hypersensitivity (DTH) |

**FIG. 6**

MHC-P
embedding

TCR binding
positive

242

**FIG. 7**

232

Concat

231

scaled dot-product attention    h

$V_{MHC}$        $K_{MHC}$        $Q_P$

230

**FIG. 8**

**FIG. 9**

**FIG. 10**

310         320         330

| first step | → | second step | → | third step |

positive      positive ←    positive

(a)

| first step | → | second step | → | third step |

negative → negative  → negative

(b)

**FIG. 11**

S1101

| generating embedding vector for first input data and second input data |

S1102

| combining grouping experimental method and experimental result data of the experimental method according to a plurality of the predetermined categories, with embedding vector |

S1103

| generating output data |

| | Europäisches Patentamt European Patent Office Office européen des brevets | **EUROPEAN SEARCH REPORT** | **Application Number** EP 24 20 8105 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/122690 A1 (LIU KAI [US] ET AL) 21 April 2022 (2022-04-21) * Paragraphs 3, 77, 96, 104-108, 157, 207, 212, 213, 220, 221, 240, 241, 260, and 269; figures 1-3,4A-4C,6,8-11 * ----- | 1-15 | INV. G16B15/30 G06N3/045 G16B40/20 G16B40/30 |
| | | | **TECHNICAL FIELDS SEARCHED (IPC)** G16B G06N |

The present search report has been drawn up for all claims

| Place of search | Date of completion of the search | Examiner |
|---|---|---|
| Munich | 19 March 2025 | Hendrikse, Natalie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 20 8105

This annex lists the patent family members relating to the patent documents cited in the above-mentioned European search report.
The members are as contained in the European Patent Office EDP file on
The European Patent Office is in no way liable for these particulars which are merely given for the purpose of information.

19-03-2025

| Patent document cited in search report | Publication date | Patent family member(s) | Publication date |
|---|---|---|---|
| US 2022122690 A1 | 21-04-2022 | AU 2021308081 A1 | 17-11-2022 |
| | | BR 112023000827 A2 | 07-02-2023 |
| | | CA 3180799 A1 | 20-01-2022 |
| | | CN 115997254 A | 21-04-2023 |
| | | EP 4182924 A1 | 24-05-2023 |
| | | EP 4513509 A2 | 26-02-2025 |
| | | IL 299801 A | 01-03-2023 |
| | | JP 2023534283 A | 08-08-2023 |
| | | KR 20230042048 A | 27-03-2023 |
| | | US 2022122690 A1 | 21-04-2022 |
| | | WO 2022016125 A1 | 20-01-2022 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020190177764